# EUROPÄISCHE PATENTANMELDUNG

(11) **EP 1 872 751 A2**
(43) Veröffentlichungstag der Anmeldung: **02.01.2008**
(21) Anmeldenummer: 07108315.8
(22) Anmeldetag: 16.05.2007
(51) Int. Cl.: A61F 5/44

(54) **Harnauffangbeutel**

(30) Priorität: 30.06.2006 DE 202006010200 U
(71) Anmelder: Grundke, Reinhold, 84547 Emmerting (DE); Hofstetter, Alfons, Prof.Dr.med., 82008 Unterhaching (DE); Lungauer, Elke, 84577 Tüssling (DE)
(72) Erfinder: Grundke, Reinhold, 84547 Emmerting (DE); Hofstetter, Alfons, Prof.Dr.med., 82008 Unterhaching (DE); Lungauer, Elke, 84577 Tüssling (DE)
(74) Vertreter: Hofstetter, Alfons J.

(57) **Zusammenfassung**

Die vorliegende Erfindung betrifft einen Harnauffangbeutel mit zwei Beutelwänden (28, 38), die an ihrem Außenumfang miteinander verbunden sind und mindestens einen Beutelraum (30) flüssigkeitsdicht begrenzen sowie mindestens einem Einlassanschluss (16), der in den Beutelraum (30) mündet und einem in dem mindestens einen Beutelraum (30) angeordneten Superabsorber, wobei der Einlassanschluss (16) ein Ventil (18) zur Verhinderung eines Harnrückflusses aus dem Beutelraum (30) aufweist.

## Beschreibung

Die vorliegende Erfindung betrifft einen Harnauffangbeutel mit zwei Beutelwänden, die an ihrem Außenumfang miteinander verbunden sind und mindestens einen Beutelraum flüssigkeitsdicht begrenzen sowie mindestens einem Einlassanschluss der in den Beutelraum mündet und einem in dem mindestens einen Beutelraum angeordneten Superabsorber. Die Erfindung betrifft zudem eine Beutelhalterung für den genannten Harnauffangbeutel.

Harnauffangtaschen sind aus dem Stand der Technik bekannt. So beschreibt die DE 20 2005 008 071 U1 eine gattungsgemäße mobile Harnauffangtasche, die insbesondere als so genannter Beinbeutel ausgebildet ist. Derartige Beinbeutel werden gewöhnlich als Urinbeutel bei Personen eingesetzt, bei denen der Urin per Urinalkondom oder Katheter abgeführt werden soll. Der mindestens einer Kammer der bekannten Harnauffangtasche angeordnete Superabsorber wird durch die Aufnahme von Urin in einen geleeartigen Zustand umgewandelt. Die auf diese Weise gelierte Masse verliert aufgrund ihrer erhöhten Zähigkeit beziehungsweise verminderten Fließfähigkeit ihre Fähigkeit zur schnellen, ungehinderten Verlagerung innerhalb des Taschenraums. Eine unkontrollierte Verlagerung des Urins innerhalb des Beutelraums wird dadurch verhindert.

Nachteilig an den bekannten Harnauffangtaschen ist jedoch, dass die Infektionsgefahr bei zu langem Tragen relativ hoch ist, da nicht ausgeschlossen werden kann, dass es zu einer Überfüllung des Taschenraums, bedingt durch ein Überschreiten der Aufnahmekapazität des Superabsorbers und dadurch zu einem Rückfluss von Harn in den Urogenitalbereich kommt. Zudem besteht die Gefahr, dass bei einem zu langen Tragen Bakterien aus dem Taschenraum in den Urogenitalbereich aufsteigen und es so zu Infektionen kommt.

Entsprechende Probleme treten auch bei so genannten Bettbeuteln auf. Bei Bettbeuteln handelt es sich um Harnauffangbeutel, die lösbar an einem Patientenbett befestigt sind. Auch hier muss vermieden werden, dass es zu einem Harnrückfluss oder zu einem Aufstieg von Bakterien aus dem Beutel in den Urogenitaltrakt kommt. Des Weiteren tritt bei derartigen Bettbeuteln das Problem auf, dass zur Leerung der bekannten Bettbeutel diese ein Auslassventil aufweisen. Der Auslass von Urin bedingt jedoch bei Beutelvolumen von ca. 1500 ml einen erheblichen Zeitaufwand für das Pflegepersonal. Zudem ergibt sich eine erhebliche Geruchsbelästigung durch den offen ablaufenden Urin.

Es ist daher Aufgabe der vorliegenden Erfindung, einen Harnauffangbeutel der eingangs genannten Art bereitzustellen, der einen Rückfluss von Harn aus dem Beutel sicher verhindert und ein Infektionsrisiko minimiert.

Es ist weiterhin Aufgabe der vorliegenden Erfindung, eine Beutelhalterung für den Harnauffangbeutel der eingangs genannten Art bereitzustellen, die einen einfachen und schnellen Wechsel von Harnauffangbeutel gewährleistet.

Zur Lösung dieser Aufgaben dienen ein gattungsgemäßer Harnauffangbeutel gemäß den Merkmalen des Anspruchs 1 und eine Beutelhalterung gemäß den Merkmalen des Anspruchs 5.

Vorteilhafte Ausgestaltungen sind in den jeweiligen Unteransprüchen beschrieben.

Ein erfindungsgemäßer Harnauffangbeutel weist zwei Beutelwände auf, die an ihrem Außenumfang miteinander verbunden sind und mindestens einen Beutelraum flüssigkeitsdicht begrenzen. Des Weiteren ist mindestens ein Einlassanschluss ausgebildet, der in den Beutelraum mündet, wobei in dem mindestens einen Beutelraum ein Superabsorber angeordnet ist. Erfindungsgemäß weist der Einlassanschluss ein Ventil zur Verhinderung eines Harnrückflusses aus dem Beutelraum auf. Dabei kann das Ventil ein Rückschlagventil, insbesondere ein so genanntes Flatterventil sein. Durch diese erfindungsgemäße Maßnahme ist gewährleistet, dass der Rückfluss von Harn aus dem Beutel in Richtung des Urogenitaltrakts sicher verhindert wird. Dadurch wird das Infektionsrisiko erheblich minimiert, ein Aufstieg von Bakterien aus dem Harnauffangbeutel in den Urogenitaltrakt über eine katheterartige Verbindung zwischen dem Einlassanschluss und dem Urogenitaltrakt wird nahezu ausgeschlossen. Zudem ist gewährleistet, dass volle Harnauffangbeutel zeitgerecht gewechselt werden, da ein Befüllen des Beutelraums nach Erreichen der maximalen Kapazität des Superabsorbers beziehungsweise des Beutelraums nicht mehr möglich ist. Ein Beutelwechsel ist zu diesem Zeitpunkt zwingend erforderlich.

In einer vorteilhaften Ausgestaltung der Erfindung ist der Superabsorber ein pulverförmiges Polymer, welches nach Aufnahme von Harn zu einer gelartigen Substanz aufquillt. Dabei wird der Superabsorber in einer Menge verwendet, die ein Gelieren des vollen Beutelinhalts gewährleistet. Insbesondere kann als Superabsorber der in der Verpackungsindustrie bekannte Superabsorber FAVOR-PACK^{®} der Firma Degussa verwendet werden, dessen Grundlage wasserunlösliche vernetzte Polymere bilden, die in der Lage sind, unter Quellung und Ausbildung formstabiler Gele große Mengen wässriger Flüssigkeiten zu absorbieren. Für ein Beutelvolumen von ca. 200 ml kann bereits 2,5 g des Superabsorbers ausreichend sein, während für ein Beutelvolumen von ca. 400 ml entsprechend 5 g des Superabsorbers benötigt werden.

In einer weiteren vorteilhaften Ausgestaltung des erfindungsgemäßen Harnauffangbeutels weist dieser eine Beutelhalterung auf, in die der Harnauffangbeutel einsteckbar ist. Die Beutelhalterung umfasst dabei erfindungsgemäß mindestens eine Aufnahmetasche zur Aufnahme des Harnauffangbeutels. Durch diese Ausgestaltung der Beutelhalterung ist erfindungsgemäß ein einfacher und schneller Wechsel von Harnauffangbeuteln gewährleistet. Ein Wechseln von vollen Harnauffangbeuteln kann im Vergleich zu einem üblichen Ablassen des Urins aus dem Beutel erheblich schneller durchgeführt werden. Zudem treten keine Geruchsbelästigungen durch offenen Urin auf. Des Weiteren ergibt sich durch die Kombination des erfindungsgemäßen Harnauffangbeutels mit der erfindungsgemäßen Beutelhalterung der Vorteil, dass der Harnauffangbeutel aus einer einfach ausgebildeten Hülle, insbesondere bestehend aus einem Kunststoff, ausgebildet sein kann, da es sich um ein "Wegwerfprodukt" handelt. Auch die Tatsache, dass kein Auslassventil mehr benötigt wird lässt die vorgenannte einfache und kostengünstige Konstruktion des erfindungsgemäßen Harnauffangbeutels zu.

In einer vorteilhaften Ausgestaltung ist die erfindungsgemäße Beutelhalterung verschließbar ausgebildet. Dabei kann insbesondere ein oberer Abschnitt der Aufnahmetasche einen Klappenabschnitt aufweisen, wobei der Klappenabschnitt mit einer ihm gegenüberliegenden Rückwand der Beutelhalterung lösbar verschließbar ausgebildet ist. Der Klappenabschnitt kann zum Beispiel mittels eines zumindest teilweise randlich umlaufenden Klett- oder Klebeverschlusses mit der Rückwand verschließbar ausgebildet sein. Dadurch wird einerseits eine sichere Halterung des Harnauffangbeutels in der Beutelhalterung und andererseits die Möglichkeit eines schnellen Wechsels des Harnauffangbeutels gewährleistet.

In einer weiteren vorteilhaften Ausgestaltung der erfindungsgemäßen Beutelhalterung weist diese mindestens eine Befestigungsvorrichtung zur lösbaren Befestigung der Beutelhalterung an einem Patientenbett oder ähnlichem auf. Die Beutelhalterung kann aber auch mindestens eine Befestigung zur lösbaren Befestigung der Beutelhalterung an oder in einer Innentasche einer Hose im Oberschenkelbereich eines Patienten aufweisen. Damit ist auch eine sichere und zudem lösbare Befestigung der Beutelhalterung in verschiedenen Einsatzbereichen gewährleistet.

In einer weiteren vorteilhaften Ausgestaltung der erfindungsgemäßen Beutelhalterung weist diese in dem der Aufnahmetasche gegenüberliegenden oberen Bereich einen taschenartig ausgebildeten Kragenabschnitt zur sicheren Verankerung des Harnauffangbeutels auf. Der taschenartig ausgebildete Kragenabschnitt bildet eine zusätzliche Möglichkeit der Sicherung des Harnauffangbeutels in der Beutelhalterung.

In einer weiteren vorteilhaften Ausgestaltung der Beutelhalterung ist in dem der Aufnahmetasche gegenüberliegenden Bereich mindestens eine Öffnung zum Durchtritt und zur Befestigung des mindestens einen Einlassanschlusses ausgebildet. Durch die Ausbildung dieser Öffnung wird eine optimale Lage und Anordnung des Einlassanschlusses relativ zur Beutelhalterung gewährleistet, so dass eine sichere Verankerung des Harnauffangbeutels in der Beutelhalterung gewährleistet ist. Dabei kann die Öffnung durch einen zwischen der Rückwand und einer Vorderwand der Beutelhalterung, die an ihren Außenumfängen miteinander verbunden sind, ausgebildeten Durchlass gebildet sein, wobei bei dieser Ausgestaltung die Aufnahmetasche und der Klappenabschnitt in der genannten Vorderwand ausgebildet sind. Es ist aber auch möglich, dass die Öffnung durch ein auf einer Vorderwand der Beutelhalterung beziehungsweise der Vorderseite der Rückwand ausgebildetes schlaufenartiges Element gebildet ist.

Weitere Einzelheiten, Merkmale und Vorteile der Erfindung ergeben sich aus einem in den folgenden Zeichnungen dargestelltem Ausführungsbeispiel. Es zeigen:
- Figur 1: eine schematische Darstellung eines erfindungsgemäßen Harnauffangbeutels;
- Figur 2: eine Schnittansicht des erfindungsgemäßen Harnauffangbeutels entlang der Linie II - II gemäß Figur 1; und
- Figur 3: eine schematische Darstellung einer erfindungsgemäßen Beutelhalterung für den erfindungsgemäßen Harnauffangbeutels.

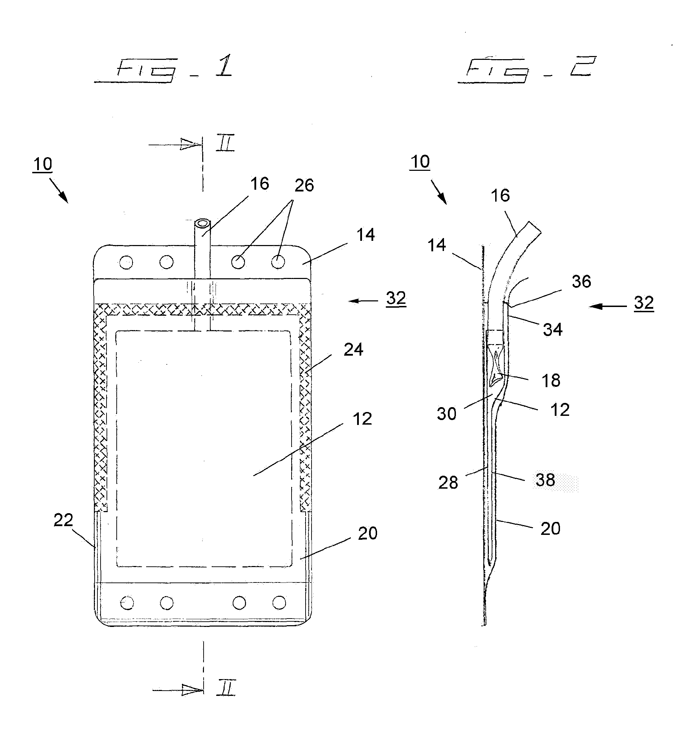

Figur 1 zeigt eine schematische Darstellung eines Harnauffangbeutels 12, wobei der Harnauffangbeutel 12 zwei Beutelwände 26, 28 aufweist, die an ihrem Außenumfang miteinander verbunden, insbesondere verschweißt sind. Der Harnauffangbeutel 12 besteht üblicherweise aus Kunststoff oder einem anderen geeigneten Plastikmaterial. Weitere Materialien sind aber ebenfalls denkbar. Der Harnauffangbeutel 12 weist zudem einen Beutelraum 30 auf, der durch die miteinander verbundenen Beutelwände 28, 38 flüssigkeitsdicht begrenzt wird. Des Weiteren ist ein Einlassanschluss 16, der in den Beutelraum 30 mündet, ausgebildet. In dem Beutelraum 30 ist ein so genannter Superabsorber angeordnet (nicht dargestellt).

Man erkennt, dass der Harnauffangbeutel 12 in einer Beutelhalterung 10 angeordnet ist. Dabei weist die Beutelhalterung 10 eine Aufnahmetasche 20 zur Aufnahme des Harnauffangbeutels 12 auf. Zudem wird deutlich, dass die Beutelhalterung 10 verschließbar ausgebildet ist. Dabei weist ein oberer Abschnitt 32 der Aufnahmetasche 20 einen Klappenabschnitt 34 auf, der wiederum mit einer ihm gegenüberliegenden Rückwand 14 der Beutelhalterung 10 lösbar verschließbar ausgebildet ist. Bei dem dargestellten Ausführungsbeispiel ist der Klappenabschnitt 34 mittels eines teilweise randlich umlaufenden Klettverschlusses 24 mit der Rückwand 14 verschließbar ausgebildet.

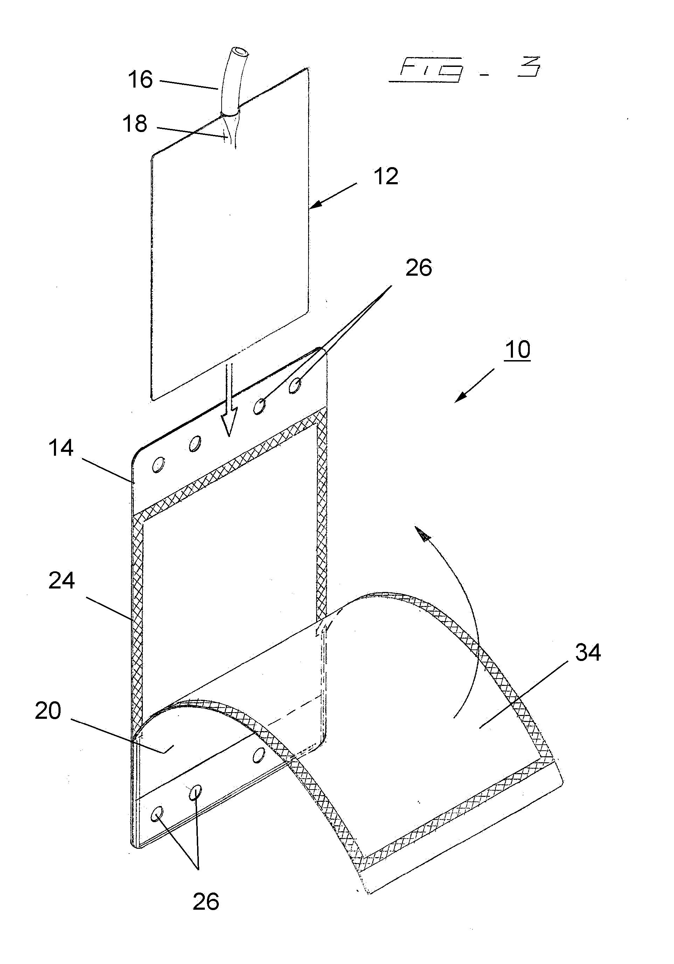

Die Ausgestaltung des Klappenabschnittes 34 beziehungsweise der Beutelhalterung 10 wird auch aus Figur 2 deutlich. Dabei zeigt die Figur 2 eine Schnittansicht des Harnauffangbeutels 12 und der Beutelhalterung 10 entlang der Linie II bis II gemäß Figur 1. Man erkennt deutlich die Anordnung beziehungsweise Lage des Harnauffangbeutels 12 innerhalb der Beutelhalterung 10 und insbesondere innerhalb der Aufnahmetasche 20. Des Weiteren erkennt man, dass der Klappenabschnitt 34 im Bereich des Einlassanschlusses 16 im geschlossenen Zustand mit der Rückwand 14 eine Öffnung 36 ausbildet, durch die der Einlassanschluss 16 führbar ist.

An dem in dem Beutelraum 30 ragenden Ende des Einlassanschlusses 16 ist ein Ventil 18 zur Verhinderung eines Harnrückflusses aus dem Beutelraum 30 in den Einlassanschluss 16 und einem damit verbundenen Katheter (nicht dargestellt) zu verhindern. In dem dargestellten Ausführungsbeispiel ist das Ventil 18 als Flatterventil ausgebildet. Auch andere Typen von Ventilen sind möglich, wobei immer gewährleistet sein muss, dass kein Flüssigkeitsdurchfluss in Richtung des Einlassanschlusses 16 erfolgen darf.

Figur 3 zeigt eine weitere schematische Darstellung der Beutelhalterung 10 für den Harnauffangbeutel 12. Man erkennt, dass der Harnauffangbeutel 12 durch ein Einstecken in eine Aufnahmetasche 20 der Beutelhalterung 10 in dieser fixiert werden kann. Der Klappenabschnitt 34 ist leicht zu öffnen, so dass ein einfacher Wechsel der Harnauffangbeutel 12 gewährleistet ist. Andererseits ist durch den Klettverschluss 24 ein sicherer Verschluss und damit eine sichere Halterung des Harnauffangbeutels 12 in der Beutelhalterung 10 gewährleistet.

Des Weiteren erkennt man, dass die Beutelhalterung 10 mehrere Befestigungsvorrichtungen 26 zur lösbaren Befestigung der Beutelhalterung an zum Beispiel einem Patientenbett aufweist (siehe auch Figur 1). Verschiedene Arten von Befestigungsvorrichtungen 26 sind hierbei denkbar, in dem dargestellten Ausführungsbeispiel sind die Befestigungsvorrichtungen 26 als einfache Öffnungen ausgebildet, so dass die Beutelhalterung 10 zum Beispiel in hakenförmige Vorsprünge des Patientenbetts eingehängt werden kann.

In einem weiteren, nicht dargestellten Ausführungsbeispiel ist es möglich, dass die Öffnung 36, welche zum Durchtritt und zur Befestigung des Einlassanschlusses 16 dient, durch einen zwischen der Rückwand 14 und einer Vorderwand 22 der Beutelhalterung 10 ausgebildeten Durchlass gebildet ist. Dabei sind die Rückwand 14 und die Vorderwand 22 an ihren Außenumfängen miteinander verbunden, insbesondere miteinander verschweißt oder verklebt. Bei diesem Ausführungsbeispiel sind die Aufnahmetasche 20 und der Klappenabschnitt 34 in der Vorderwand 22 ausgebildet.

## Patentansprüche

1. Harnauffangbeutel mit zwei Beutelwänden (28, 38), die an ihrem Außenumfang miteinander verbunden sind und mindestens einen Beutelraum (30) flüssigkeitsdicht begrenzen sowie mindestens einem Einlassanschluss (16) der in den Beutelraum (30) mündet und einem in dem mindestens einen Beutelraum (30) angeordneten Superabsorber,
**dadurch gekennzeichnet,**
**dass** der Einlassanschluss (16) ein Ventil (18) zur Verhinderung eines Harnrückflusses aus dem Beutelraum (30) aufweist.

2. Harnauffangbeutel nach Anspruch 1,
**dadurch gekennzeichnet,**
**dass** das Ventil (18) ein Rückschlagventil, insbesondere ein Flatterventil ist.

3. Harnauffangbeutel nach Anspruch 1 oder 2,
**dadurch gekennzeichnet,**
**dass** der Superabsorber ein pulverförmiges Polymer ist, welches nach Aufnahme von Harn zu einer gelartigen Substanz aufquillt.

4. Harnauffangbeutel nach einem der vorhergehenden Ansprüche,
**dadurch gekennzeichnet,**
**dass** der Harnauffangbeutel (12) eine Beutelhalterung (10) aufweist in die der Harnauffangbeutel (12) einsteckbar ist.

5. Beutelhalterung für einen Harnauffangbeutel (12) nach einem der Ansprüche 1 bis 4,
**dadurch gekennzeichnet,**
**dass** die Beutelhalterung (10) mindestens eine Aufnahmetasche (20) zur Aufnahme des Harnauffangbeutels (12) aufweist.

6. Beutelhalterung nach Anspruch 5,
**dadurch gekennzeichnet,**
**dass** die Beutelhalterung (10) verschließbar ausgebildet ist.

7. Beutelhalterung nach Anspruch 6,
**dadurch gekennzeichnet,**
**dass** ein oberer Abschnitt (32) der Aufnahmetasche (20) einen Klappenabschnitt (34) aufweist, wobei der Klappenabschnitt (34) mit einer ihm gegenüberliegenden Rückwand (14) der Beutelhalterung (10) lösbar verschließbar ausgebildet ist.

8. Beutelhalterung nach Anspruch 7,
**dadurch gekennzeichnet,**
**dass** der Klappenabschnitt (34) mittels eines zumindest teilweise randlich umlaufenden Klett- oder Klebeverschlusses (24) mit der Rückwand (14) verschließbar ausgebildet ist.

9. Beutelhalterung nach einem der Ansprüche 5 bis 8,
**dadurch gekennzeichnet,**
**dass** die Beutelhalterung (10) mindestens eine Befestigungsvorrichtung (26) zur lösbaren Befestigung der Beutelhalterung (10) an einem Patientenbett oder Ähnlichem aufweist.

10. Beutelhalterung nach einem der Ansprüche 5 bis 8,
**dadurch gekennzeichnet,**
**dass** die Beutelhalterung (10) mindestens eine Befestigungsvorrichtung zur lösbaren Befestigung der Beutelhalterung (10) an oder in einer Innentasche einer Hose im Oberschenkelbereich eines Patienten aufweist.

11. Beutelhalterung nach einem der Ansprüche 5 bis 10,
**dadurch gekennzeichnet,**
**dass** die Beutelhalterung (10) in dem der Aufnahmetasche (20) gegenüberliegenden oberen Bereich einen taschenartig ausgebildeten Kragenabschnitt zur sicheren Verankerung des Harnauffangbeutels (12) aufweist.

12. Beutelhalterung nach einem der Ansprüche 5 bis 11,
**dadurch gekennzeichnet,**
**dass** die Beutelhalterung (10) in dem der Aufnahmetasche (20) gegenüberliegenden oberen Bereich mindestens eine Öffnung (36) zum Durchtritt und zur Befestigung des mindestens einen Einlassanschlusses (16) aufweist.

13. Beutelhalterung nach Anspruch 12,
**dadurch gekennzeichnet,**
**dass** die Öffnung (36) durch einen zwischen der Rückwand (14) und einer Vorderwand (22) der Beutelhalterung (10), die an ihren Außenumfängen miteinander verbunden sind, ausgebildeten Durchlass gebildet ist und die Aufnahmetasche (20) und der Klappenabschnitt (34) in der Vorderwand (22) ausgebildet sind.

14. Beutelhalterung nach Anspruch 12,
**dadurch gekennzeichnet,**
**dass** die Öffnung (36) durch ein auf einer Vorderwand (22) oder einer Vorderseite der Rückwand (14) der Beutelhalterung (10) ausgebildetes schlaufenartiges Element gebildet ist.
